# EUROPEAN PATENT APPLICATION

(11) **EP 2 218 466 A1**
(43) Date of publication of application: **18.08.2010**
(21) Application number: 08854298.0
(22) Date of filing: 21.11.2008
(51) Int. Cl.: A61L 27/00, A61F 2/02

(54) **LACTIDE/ -CAPROLACTONE COPOLYMER FOR MEDICAL IMPLANT, METHOD FOR PRODUCING LACTIDE/ -CAPROLACTONE COPOLYMER FOR MEDICAL IMPLANT, MEDICAL IMPLANT AND ARTIFICIAL DURA MATER**

(30) Priority: 29.11.2007 JP 2007308547; 29.11.2007 JP 2007308548
(71) Applicant: Gunze Limited, Ayabe-shi, Kyoto 623-8511 (JP)
(72) Inventor: ARIMURA, Hidetoshi, Ayabe-shi Kyoto 623-8512 (JP); TAIRA, Tsuguyoshi, Ayabe-shi Kyoto 623-8512 (JP); TAKAHASHI, Yoshitake, Ayabe-shi Kyoto 623-8512 (JP)
(74) Representative: Beckmann, Claus
(86) International application number: PCT/JP2008/071249
(87) International publication number: WO 2009/069558

(57) **Abstract**

An object of the present invention is to provide a flexible lactide-ε-caprolactone copolymer for a medical implant that is extremely safe as a result of not containing higher alcohol and is useful for fabricating medical implants requiring flexibility, such as artificial dura mater and artificial blood vessels. In addition, an object of the present invention is to provide a method for producing the lactide-ε-caprolactone copolymer for a medical implant, a medical implant and an artificial dura mater which are obtained by using the lactide-ε-caprolactone copolymer for a medical implant.

The present invention is a lactide-ε-caprolactone copolymer for a medical implants, wherein the molar ratio of a lactide to ε-caprolactone (lactide/ε-caprolactone) is 40/60 to 60/40, the weight average molecular weight is 100000 to 500000, a higher alcohol component is not contained therein, and the fusion enthalpy derived from a crystal when formed into a molding by heating and melting is 10 J/g or less.

## Description

### TECHNICAL FIELD

The present invention relates to a flexible lactide-ε-caprolactone copolymer for a medical implant that is extremely safe as a result of not containing higher alcohol and is useful for fabricating medical implants requiring flexibility, such as artificial dura mater and artificial blood vessels. In addition, the present invention relates to a method for producing the lactide-ε-caprolactone copolymer for a medical implant, a medical implant and an artificial dura mater which are obtained by using the lactide-ε-caprolactone copolymer for a medical implant.

### BACKGROUND ART

Biodegradable/absorbable polymers such as polylactides have come to be frequently used as medical implants composed of polymer materials in recent years. Since biodegradable/absorbable polymers gradually lose their shape due to hydrolysis, they are not required to be removed by additional surgery in the manner of conventional non-degradable medical implants, thereby making it possible to considerably reduce the burden on patients.

For example, dura mater, which is present between the skull and brain and covers the spinal cord, mainly fulfills the function of protecting the brain and spinal cord as well as preventing leakage of cerebrospinal fluid. In the case of using a procedure involving an incision of the dura mater during neurosurgical operation, it is necessary to replace missing or contracted dura mater. Freeze-dried human dura mater has conventionally been used for this replacement material. However, the human dura mater used for this purpose has problems with product uniformity and ease of supply. Moreover, there has been a report (Non-patent Document 1) of the possibility of infection of Creutzfeldt-Jakob disease by human dura mater, and a notification prohibiting the use of human dura mater was released by the Japanese Ministry of Health and Welfare on April 7, 1997. With this in mind, biodegradable/absorbable synthetic polymers, and particularly artificial dura mater composed of lactide-ε-caprolactone copolymer, have been proposed (Patent Document 1). In addition to being nearly completely free of risk of infection, this type of artificial dura mater has strength to a degree that allows the obtaining of adequate suture strength, and is able to prevent problems associated with long-term implantation since it decomposes after fulfilling its role during a fixed period of time.

Examples of the above-mentioned biodegradable/absorbable polymers include aliphatic polyesters such as polyglycolides, polylactides, polycaprolactones or polyvalerolactones, as well as polyester ethers (poly-1,4-dioxanone-2-one, poly-1,5-dioxepane-2-one, copolymers of ethylene glycol and the above-mentioned aliphatic polyesters), copolymers of the above-mentioned aliphatic polyesters and polyester ethers, amino acids, hyaluronic acid, alginic acid and oxidized cellulose. In particular, since lactide-ε-caprolactone copolymers also have comparatively superior flexibility, they are attracting attention as materials of medical implants requiring flexibility, such as artificial dura mater and artificial blood vessels (for example, Patent Document 2).

However, although lactide-ε-caprolactone copolymers have high flexibility immediately after polymerization, they have the problem of gradually losing flexibility if subsequently left alone (to be referred to as "self-setting"). In particular, a molding is ordinarily formed by heating and melting in order to form a medical implant, and this loss of flexibility is prominent following heating and melting.

Patent Document 3 describes a bioabsorbable polymer composed of a copolymer of a lactide and ε-caprolactone in which there is hardly any occurrence of self-setting. The invention described in Patent Document 3 inhibits the progression of self-setting by adjusting the average chain length of the lactide and ε-caprolactone in the copolymer, and describes that average chain length is adjusted with the reaction temperature during copolymer production, and that an average chain length that most effectively inhibits progression of self-setting is obtained at a reaction temperature of about 140°C.

However, when an experiment was carried out in Patent Document 3 that was premised on the use of a higher alcohol as a polymerization initiator during polymerization of the copolymer of a lactide and ε-caprolactone, there was the problem of encountering hesitation when considering the use of the copolymer as a medical implant since the higher alcohol remains after modifying the end of the copolymer molecular chain.
Patent Document 1: Japanese Kokai Publication Hei-8-80344 (JP-A H08-80344)
Patent Document 2: Japanese Kohyo Publication Hei-6-501045 (JP-T H06-501045)
Patent Document 3: Japanese Kokai Publication 2005-306999 (JP-A 2005-306999)
Non-patent Document 1: Neurosurgery (in Japanese), 21(2), 167-170, 1993

### DISCLOSURE OF THE INVENTION

### PROBLEMS TO BE SOLVED BY THE INVENTION

An object of the present invention is to provide a flexible lactide-ε-caprolactone copolymer for a medical implant that is extremely safe as a result of not containing higher alcohol and is useful for fabricating medical implants requiring flexibility, such as artificial dura mater and artificial blood vessels. In addition, an object of the present invention is to provide a method for producing the lactide-ε-caprolactone copolymer for a medical implant, a medical implant and an artificial dura mater which are obtained by using the lactide-ε-caprolactone copolymer for a medical implant.

### MEANS FOR SOLVING THE PROBLEMS

The present invention is a lactide-ε-caprolactone copolymer for a medical implant, wherein the molar ratio of a lactide to ε-caprolactone (lactide/ε-caprolactone) is 40/60 to 60/40, the weight average molecular weight is 100000 to 500000, a higher alcohol component is not contained therein, and the fusion enthalpy derived from a crystal when formed into a molding by heating and melting is 10 J/g or less.
The following provides a detailed description of the present invention.

During polymerization of a copolymer of a lactide and ε-caprolactone, it has been a matter of technical common sense that is essential to add a higher alcohol as a polymerization initiator in order to obtain a copolymer having a high degree of polymerization while avoiding uneven polymerization caused by differences in reactivity between the lactide and ε-caprolactone. However, as a result of conducting extensive studies, the inventors of the present invention found that, in the case of carrying out a copolymerization reaction at a high temperature above 130°C, a copolymer having an adequate degree of polymerization can be obtained without using a higher alcohol as the polymerization initiator. It was also found that a copolymer obtained by reacting at a high temperature above 130°C without using a higher alcohol as the polymerization initiator not only has a higher degree of safety as a result of not containing higher alcohol, but also demonstrates high flexibility without the occurrence of self-setting even after being formed into a molding by heating and melting, thereby leading to completion of the present invention.

The lactide-ε-caprolactone copolymer for a medical implant (to also be simply referred to as the "copolymer") of the present invention is a copolymer of a lactide and ε-caprolactone.
In the copolymer of the present invention, the molar ratio of the lactide to ε-caprolactone (lactide/ε-caprolactone) is 40/60 to 60/40. If the molar ratio of either the lactide or ε-caprolactone exceeds 60 mol%, crystallinity of the copolymer increases and the copolymer hardens, thereby preventing it from being used in medical implants requiring flexibility, such as artificial dura mater and artificial blood vessels. The molar ratio of the lactide to ε-caprolactone is more preferably 45/55 to 55/45.

The lower limit of weight average molecular weight of the copolymer of the present invention is 100000, while the upper limit is 500000. If the weight average molecular weight is less than 100000, a molding (medical implant) having adequate strength is unable to be obtained. If the weight average molecular weight exceeds 500000, melt viscosity of the copolymer increases resulting in inferior moldability. The lower limit of the weight average molecular weight is preferably 150000 and the upper limit is preferably 450000.

The copolymer of the present invention does not contain a higher alcohol component. In the case of containing a higher alcohol component, there is concern over effects on the human body depending on the concentration thereof, thereby making use as a medical implant difficult.

The fusion enthalpy of the copolymer of the present invention derived from a crystal when formed into a molding by heating and melting is 10 J/g or less. If the fusion enthalpy exceeds 10 J/g, crystallinity of the molding increases and the molding hardens, and this can cause problems such as inducing inflammation due to damage to soft tissue caused by so-called compliance mismatching in which the molding is unable to follow deformation or stretching of the soft tissue, thereby preventing use as medical implants requiring flexibility, such as artificial dura mater and artificial blood vessels. The lower limit of the above-mentioned fusion enthalpy is preferably 1 J/g. If the fusion enthalpy is less than 1 J/g, mechanical strength of the molding may considerably decrease or dimensions of the molding may change during storage.

In the present description, fusion enthalpy derived from a crystal refers to the amount of heat per unit weight measured when a biodegradable/absorbable polymer is measured with a differential scanning calorimeter (DSC), and indicates the value of the first scan. The degree of crystallinity can be said to be larger the larger value of this fusion enthalpy.
A copolymer of the lactide and ε-caprolactone can be made to have a fusion enthalpy of 10 J/g or less, namely to be in a poorly crystallized state, comparatively easily immediately after polymerization. However, in order to produce a medical implant using this copolymer, it is necessary to form a molding by heating and melting. In the case of conventional copolymers of a lactide and ε-caprolactone, going through such a processing step causes fusion enthalpy derived from a crystal to increase prominently (namely, causes the copolymer to crystallize), thereby resulting in a loss of flexibility.
The fusion enthalpy derived from a crystal of the copolymer of the present invention, even after being subjected to a heating and melting step, is 10 J/g or less, or in other words, the change in fusion enthalpy derived from a crystal is extremely small.

In the method for producing the copolymer of the present invention, it is important to: 1) not add a higher alcohol as a polymerization initiator, and 2) use a polymerization temperature above 130°C, during copolymerization of a conventionally known lactide and ε-caprolactone.
Not adding a higher alcohol makes it possible to reliably prevent the resulting copolymer from containing a higher alcohol component. Although a higher alcohol was considered to be an essential component according to conventional technical common sense, problems resulting from not containing higher alcohol do not occur as a result of using a reaction temperature above 130°C. Moreover, the use of a reaction temperature above 130°C makes it possible to obtain a resin having extremely small changes in fusion enthalpy derived from a crystal. The preferable lower limit of the reaction temperature is 135°C. The reaction temperature is not particularly limited, and the preferable upper limit is 170°C. If the reaction temperature exceeds 170°C, undesirable properties such as coloration may appear in the resulting copolymer.

The activation energy required for ring-opening varies considerably between the lactide and ε-caprolactone, with the activation energy required for ring-opening being lower for the lactide than for the ε-caprolactone. Namely, the lactide is polymerized preferentially the lower the polymerization temperature, resulting in increased chain length of the lactide and higher crystallinity of the copolymer. On the other hand, the chain lengths of the lactide and ε-caprolactone become shorter the higher the polymerization temperature, and each monomer is randomly arranged in the molecular chain, thereby making it possible to lower crystallinity the copolymer. As a result of using a reaction temperature above 130°C, the crystallinity of the resulting copolymer can be lowered and crystallization can be prevented from proceeding significantly even if going through a processing step such as heating and melting.
A method for producing a lactide-ε-caprolactone copolymer for a medical implant by copolymerizing a lactide and ε-caprolactone, wherein a higher alcohol is not added as a polymerization initiator, with a reaction temperature above 130°C, is also one aspect of the present invention.

During the above-mentioned copolymerization of the lactide and ε-caprolactone, an organometallic compound such as that of tin, zinc, iron, nickel, aluminum, potassium, sodium, titanium, antimony or bismuth, or a salt thereof, may be used as a polymerization catalyst. In particular, tin compounds are preferable from the viewpoints of usage results and ease of handling during polymerization, while tin octylate is more preferable from the viewpoints of reaction rate and stability.

In order to use in medical implant applications, metal compounds that are harmful to the human body are preferably not contained whenever possible. For example, although tin octylate is known to have comparatively low toxicity with respect to living organisms, the effects thereof are considered to be extremely small particularly at a level of less than 1 ppm.
In the case of using the polymerization catalyst during the polymerization of the lactide and ε-caprolactone, it is preferable to remove the metal catalyst from the copolymer following polymerization.

The method used to remove the metal catalyst is not particularly limited, and a method that uses a catalyst remover, for example, is both simple and effective.
As the catalyst remover, a catalyst remover composed of an organic acid and alcohol is preferable, and in particular, that composed of acetic acid for the organic acid and isopropanol for the alcohol is preferable in terms of safety and ease of recovery. Here, the composite ratios of the organic acid and alcohol are such that a higher composite ratio of organic acid, within a range that does not cause the copolymer to dissolve, enables the catalyst to be removed effectively.
More specifically, for example, the ratio of acetic acid/isopropanol is preferably 15/85 to 35/65 (volume ratio). If the concentration of acetic acid is less than 15% by volume, the catalyst may not be able to be removed to a level of 1 ppm or less. If the concentration of acetic acid exceeds 35% by volume, the copolymer may dissolve, making recovery difficult.

The copolymer of the present invention can be processed by heating and melting into various forms such as sheets, foamed bodies and fibrous bodies, and the copolymer loses hardly any flexibility even if subjected to such processing. Moreover, the copolymer is extremely safe as a result of not containing higher alcohol.

The copolymer of the present invention is preferable for fabricating medical implants requiring flexibility, such as artificial dura mater and artificial blood vessels. A medical implant obtained by using the lactide-ε-caprolactone copolymer for a medical implant of the present invention is also one aspect of the present invention.
Examples of forms of the medical implant of the present invention include a film, sheet, foamed body, fibrous structure, molding and composites thereof.
In addition to the artificial dura mater and artificial blood vessels described above, the medical implant of the present invention is also preferable for use as sutures, bone cement, fracture immobilization materials, tissue replacement materials, tissue strengthening materials, tissue covering materials, tissue regeneration scaffold materials, tissue prosthetic materials, adhesion prevention materials, artificial valves, stents, clips, fiber cloth, hemostats, adhesives and coating agents.

The copolymer of the present invention is highly flexible and does not exhibit self-setting. An artificial dura mater, having a sheet composed of the copolymer of the present invention for the outermost layer, and a reinforcing layer composed of a fibrous structure capable of realizing high suture strength for the inner layer, is capable of demonstrating high flexibility and mechanical strength capable of withstanding suturing.
An artificial dura mater having a multilayer structure composed of a biodegradable/absorbable synthetic polymer, which has an outermost layer composed of the lactide-ε-caprolactone copolymer for a medical implant of the present invention and a reinforcing layer having a fibrous structure composed of a biodegradable/absorbable polymer, is also one aspect of the present invention.

The artificial dura mater of the present invention has an outermost layer composed of the copolymer of the present invention. The artificial dura mater of the present invention is able to demonstrate high flexibility that allows it to follow subtle curvatures according to the application site as well as allow the ends thereof to adequately adhere to dura mater to prevent the occurrence of fluid leakage from between the artificial dura mater and surrounding tissue following suturing.

The lower limit of fusion enthalpy derived from a crystal of the above-mentioned outermost layer is 1 J/g while the upper limit is 10 J/g. If the fusion enthalpy is less than 1 J/g, mechanical strength of the outermost layer ends up decreasing considerably or dimensions of the outermost layer end up changing during storage. If the fusion enthalpy exceeds 10 J/g, crystallinity of the outermost layer increases and the outermost layer hardens, thereby impairing flexibility and causing damage to the brain surface, or causing the ends to become free during suturing resulting in leakage of fluid from between the artificial dura mater and surrounding tissue even if sutured.

The thickness of the outermost layer is not particularly limited, and the preferable lower limit is 50 µm and the preferable upper limit is 800 µm. If the thickness is less than 50 µm, the thickness of the entire artificial dura mater may become inadequate, resulting that strength becomes deficient and there is a risk of fluid leakage. If the thickness exceeds 800 µm, the entire artificial dura mater may become excessively thick, resulting in impaired flexibility. The lower limit of thickness is more preferably 100 µm while the upper limit is more preferably 300 µm.

The artificial dura mater of the present invention has a reinforcing layer having a fibrous structure composed of a biodegradable/absorbable polymer. The reinforcing layer constitutes one of the inner layers of the multilayer structure, and imparts high suture strength to the artificial dura mater of the present invention. In addition, the reinforcing layer also has the effect of inhibiting expansion of needle holes during suturing, thereby making it possible to effectively prevent fluid leakage from needle holes.

The biodegradable/absorbable polymer that composes the above-mentioned reinforcing layer is not particularly limited, and a biodegradable/absorbable polymer which has a higher melting point and does not dissolve in the same solvent with respect to the lactide-ε-caprolactone copolymer used in the outermost layer is preferable. In addition, in the case of selecting a transparent polymer, transparency of the entire artificial dura mater can be ensured, and internal status can be observed through the artificial dura mater during and after replacement, thereby enabling problems to be discovered promptly.
Examples of such biodegradable/absorbable polymers include polyglycolides, glycolide-ε-caprolactone copolymers, lactide-glycolide copolymers, lactide-glycolide-ε-caprolactone copolymers and polydioxanones.

The above-mentioned fibrous structure is not particularly limited, and examples thereof include cloth, woven fabric, knitted fabric, web, lace, felt and non-woven fabric.

The weight per square-meter of the above-mentioned reinforcing layer is not particularly limited, and the lower limit is preferably 15 g/m² and the upper limit is preferably 45 g/m². If the weight per square-meter is less than 15 g/m², adequate reinforcing effects may not be obtained. If the weight per square-meter exceeds 45 g/m², flexibility of the overall artificial dura mater may be impaired. More preferably, the lower limit is 20 g/m² and the upper limit is 40 g/m².

The thickness of the above-mentioned reinforcing layer is not particularly limited, and the lower limit is preferably 50 µm and the upper limit is preferably 200 µm. If the thickness is less than 50 µm, adequate reinforcing effects may not be obtained. If the thickness exceeds 200 µm, flexibility of the overall artificial dura mater may be impaired. More preferably, the lower limit is 80 µm and the upper limit is 120 µm.

In addition to the outermost layer and reinforcing layer as described above, the artificial dura mater of the present invention may also have, for example, a stretchable layer for inhibiting spinal fluid leakage from a needle hole and a hydrophilic gel layer for maintaining a moist state.

The preferable lower limit of the coefficient of static friction of the surface of the artificial dura mater of the present invention as measured according to a method in compliance with ASTM D 1894 is 1.5, while the preferable upper limit is 10. If the coefficient of static friction is less than 1.5, the surface may become slippery during suturing procedures, potentially causing damage to the brain surface. If the coefficient of static friction exceeds 10, it may become difficult to separate the artificial dura mater from each other when it has become adhered, thereby resulting in inferior manipulability.

The preferable lower limit of bending stiffness of the artificial dura mater of the present invention as measured according to a method in compliance with a KES system is 0.1 gf · cm²/cm, and the preferable upper limit is 1.0 gf · cm²/cm. If the bending stiffness is less than 0.1 gf · cm²/cm, the artificial dura mater may be bent easily by its own weight, thereby resulting in inferior manipulability. If the bending stiffness exceeds 1.0 gf · cm²/cm, the artificial dura mater may not follow the profile of the site where tissue is to be replaced, thereby damaging the area around the replaced tissue or making it difficult to suture so as to prevent fluid leakage.

The preferable lower limit of tensile strength of the artificial dura mater of the present invention as measured according to method in compliance with JIS K 7113 is 5 MPa, and the preferable upper limit is 15 MPa. If tensile strength is less than 5 MPa, the artificial dura mater may rupture easily during suturing or demonstrate other problems such as being unable to withstand intracranial pressure. If the tensile strength exceeds 15 MPa, although the risk of rupture during suturing is avoided, the artificial dura mater itself may become hard, thereby resulting in a greater risk of damaging the brain surface and tissue.

The preferable lower limit of elastic modulus of the artificial dura mater of the present invention when stretched by 10% as measured according to a method in compliance with JIS K 7113 is 10 MPa, and the preferable upper limit is 20 MPa. If the elastic modulus is less than 10 MPa, deformation at a sutured site during stretching may increase resulting in a greater risk of spinal fluid leakage. If the elastic modulus exceeds 20 MPa, the artificial dura mater may become hard and difficult to stretch, thereby possibly damaging tissue during suturing.

The method used to produce the artificial dura mater of the present invention is not particularly limited, and an example of such a method consists of separately fabricating the above-mentioned outermost layer and reinforcing layer, and integrating the two layers by vacuum-pressing while heating.
A sheet that composes the outermost layer can be prepared by, for example, melt-molding the lactide-ε-caprolactone copolymer of the present invention with an extruding machine.
A fibrous structure that composes the above-mentioned reinforcing layer can be prepared by, for example, melt-blowing the raw material in the form of a biodegradable/absorbable synthetic polymer using an extruding machine.

### EFFECT OF THE INVENTION

According to the present invention, it is possible to provide a flexible lactide-ε-caprolactone copolymer for a medical implant that is extremely safe as a result of not containing higher alcohol and is useful for fabricating medical implants requiring flexibility, such as artificial dura mater and artificial blood vessels. In addition, it is possible to provide a method for producing the lactide-ε-caprolactone copolymer for a medical implant, a medical implant and an artificial dura mater which are obtained by using the lactide-ε-caprolactone copolymer for a medical implant.

### BEST MODE FOR CARRYING OUT THE INVENTION

Although the following provides a more detailed explanation of the present invention through examples thereof, the present invention is not limited to these examples.

### (Example 1)

1440 g of L-lactide, 1140 g of ε-caprolactone and 300 ppm of tin octylate (78 ppm as tin) were placed in a separable flask and polymerized for 7 days at 135°C in a nitrogen atmosphere after reducing pressure.
The resulting copolymer was treated with a catalyst remover (acetic acid/isopropanol = 30/70 v/v) and dried in a vacuum at 40°C to obtain a lactide-ε-caprolactone copolymer.

The resulting lactide-ε-caprolactone copolymer was molded into the shape of a sheet having a thickness of 100 µm by melt-molding with an extruding machine and then further subjected to heat treatment for 12 hours at 70°C in a vacuum to obtain a sheet.

### (Example 2)

1440 g of L-lactide, 1140 g of ε-caprolactone and 300 ppm of tin 2-ethylhexanoate (78 ppm as tin) were placed in a separable flask and polymerized for 7 days at 140°C in a nitrogen atmosphere after reducing pressure.

The resulting copolymer was treated with an isopropanol solution of acetic acid (acetic acid/isopropanol = 20/80 v/v) and dried in a vacuum at 40°C to obtain a lactide-ε-caprolactone copolymer.
A sheet was obtained in the same manner as Example 1 with the exception of using the resulting lactide-ε-caprolactone copolymer.

### (Comparative Example 1)

1440 g of L-lactide, 1140 g of ε-caprolactone and 300 ppm of tin 2-ethylhexanoate (78 ppm as tin) were placed in a separable flask and polymerized for 7 days at 130°C in a nitrogen atmosphere after reducing pressure.
The resulting copolymer was treated with an isopropanol solution of acetic acid (acetic acid/isopropanol = 30/70 v/v) and dried in a vacuum at 40°C to obtain a lactide-ε-caprolactone copolymer.
A sheet was obtained in the same manner as Example 1 with the exception of using the resulting lactide-ε-caprolactone copolymer.

### (Evaluation)

The lactide-ε-caprolactone copolymers and sheets obtained in Examples 1 and 2 and Comparative Example 1 were evaluated using the methods described below.

The results are shown in Table 1.

### (1) Measurement of Weight Average Molecular Weight

The weight average molecular weights of the copolymer immediately after polymerization and immediately after catalyst removal as well as the weight average molecular weight of the sheet immediately after molding were measured by gel permeation chromatography (GPC) using chloroform for the eluent (at an elusion rate of 1 ml/min), and each of the converted molecular weights was determined by using polystyrene for the reference substance.

### (2) Measurement of Fusion Enthalpy

The fusion enthalpies of the copolymer immediately after polymerization and immediately after catalyst removal as well as the fusion enthalpy of the sheet immediately after molding were measured as a fusion enthalpy derived from a crystal with a differential scanning calorimeter (DSC, heating rate of 10°C/min).

### (3) Measurement of Residual Amount of Catalyst

The amounts of catalyst remaining in the copolymer immediately after polymerization and immediately after catalyst removal as well as the amount of catalyst remaining in the sheet immediately after molding were measured by IPC emission spectrophotometry.

**[Table 1]**

| | | Example 1 | Example 2 | Comparative Example 1 |
|---|---|---|---|---|
| Weight average molecular weight | Immediately after polymerization | 347000 | 408000 | 432000 |
| | after catalyst removal | 300000 | 297000 | 334000 |
| | Immediately after sheet molding | 240000 | 142000 | 253000 |
| Fusion enthalpy (J/g) | Immediately after polymerization | 2. 4 | Not detected | 2. 8 |
| | Immediately after catalyst removal | 3. 1 | Not detected | 7. 1 |
| | Immediately after sheet molding | 2. 0 | Not detected | 19. 4 |
| Residual amount of catalyst (ppm) | Immediately after polymerization | 78 | 79 | 78 |
| | Immediately after catalyst removal | 0. 5 | 0. 7 | 0. 5 |
| | Immediately after sheet molding | 0. 5 | 0. 7 | 0. 5 |

According to Table 1, the copolymer produced in Examples 1 or 2 exhibited little difference between fusion enthalpies measured immediately after polymerization, immediately after catalyst removal and immediately after sheet molding, and had a fusion enthalpy of 10 J/g or less even after sheet molding. On the other hand, although the copolymer produced in Comparative Example 1 exhibited fusion enthalpies of 10 J/g or less immediately after polymerization and immediately after catalyst removal, the fusion enthalpy exceeded 10 J/g after sheet molding.

### (Example 3)

### (1) Synthesis of Lactide-ε-caprolactone Copolymer

1440 g of L-lactide, 1140 g of ε-caprolactone and 300 ppm of tin 2-ethylhexanoate were placed in a separable flask and polymerized for 7 days at 135°C in a nitrogen atmosphere after reducing pressure.
The resulting lactide-ε-caprolactone copolymer was treated with an isopropanol solution of acetic acid (acetic acid/isopropanol = 30/70 v/v) and dried in a vacuum at 40°C.
The resulting copolymer had a weight average molecular weight of 320000 (as measured by GPC) and a metal content of less than 0.5 ppm (as measured by IPC emission spectrophotometry).

### (2) Preparation of Sheet for Outermost Layer

A sheet having a thickness of 100 µm was obtained by melt-molding the resulting copolymer with an extruding machine.
An 8 mg sample was cut out of the resulting sheet and the fusion enthalpy derived from a crystalline portion of the sheet was measured by DSC (DSC heating rate of 10°C/min).

### (3) Preparation of Fibrous Structure

Polyglycolic acid (intrinsic viscosity of 1.8) was melt-molded by melt-blowing using an extruding machine to prepare a non-woven fabric having a weight per square-meter of 30 g/m².

### (4) Production of Artificial Dura mater

Sheets composed of the copolymer were laminated to both sides of the resulting polyglycolic acid non-woven fabric followed by integrating into a single unit by vacuum-pressing under conditions of 150°C and 100 kg/cm² to obtain artificial dura mater having a trilayer structure (thickness of approx. 200 µm).

### (Comparative Example 2)

1440 g of L-lactide, 1140 g of ε-caprolactone and 300 ppm of tin 2-ethylhexanoate were placed in a separable flask and polymerized for 7 days at 140°C in a nitrogen atmosphere after reducing pressure.
The resulting lactide-ε-caprolactone copolymer was treated with an isopropanol solution of acetic acid (acetic acid/isopropanol = 30/70 v/v) and dried in a vacuum at 40°C.
The resulting copolymer had a weight average molecular weight of 300000 (as measured by GPC) and a metal content of less than 0.5 ppm (as measured by IPC emission spectrophotometry).
Artificial dura mater was produced in the same manner as Example 3 with the exception of using the resulting copolymer.

### (Comparative Example 3)

1440 g of L-lactide, 1140 g of ε-caprolactone and 300 ppm of tin 2-ethylhexanoate were placed in a separable flask and polymerized for 7 days at 130°C in a nitrogen atmosphere after reducing pressure.
The resulting lactide-ε-caprolactone copolymer was treated with an isopropanol solution of acetic acid (acetic acid/isopropanol = 30/70 v/v) and dried in a vacuum at 40°C.
The resulting copolymer had a weight average molecular weight of 350000 (as measured by GPC) and a metal content of less than 0.5 ppm (as measured by IPC emission spectrophotometry).
Artificial dura mater was produced in the same manner as Example 3 with the exception of using the resulting copolymer.

### (Evaluation)

The artificial dura mater produced in Example 3 and Comparative Examples 2 and 3 were evaluated using the methods described below.
The results are shown in Table 2.

### (1) Measurement of Coefficient of Static Friction

The coefficient of static fraction of the surface of the artificial dura mater was measured using a friction coefficient tester (Shinto Scientific Co., Ltd., HEIDON-14DR) according to a method in compliance with ASTM D 1894.

### (2) KES Bending Test

The artificial dura mater were respectively cut into test pieces measuring 50 mm × 100 mm, and bending stiffness was measured using a pure bending tester (KES-FB2).

### (3) Tensile Test

The artificial dura mater were respectively cut into test pieces measuring 10 mm x 80 mm, and tensile strength and elastic modulus when stretched by 10% were measured by carrying out a tensile test at a chuck interval of 40 mm and pulling rate of 50 mm/min.

**[Table 2]**

| | | Example 3 | Comparative Example 2 | Comparative Example 3 |
|---|---|---|---|---|
| Outermost layer | Copolymer polymerization (°C) temperature | 135 | 140 | 130 |
| | Fusion enthalpy (J/g) | 2. 0 | Not detected | 19. 4 |
| Evaluation | Coefficient of static friction | 5. 88 | 14. 22 | 1. 08 |
| | Bending stiffness (gf/cm²/cm) | 0. 5 | 0. 06 | 1. 8 |
| | Tensile strength (MPa) | 8. 2 | 4. 6 | 13. 1 |
| | Elastic modulus (MPa) when stretched by 10% | 15. 1 | 9. 2 | 22. 4 |

According to Table 2, the artificial dura mater produced in Example 3 exhibited fusion enthalpy derived from the crystal of the outermost layer of 2.0 J/g, and demonstrated performance required for application as artificial dura mater with respect to coefficient of static friction, bending stiffness, tensile strength and elastic modulus when stretched by 10%.
In contrast, in the case of the artificial dura mater produced in Comparative Example 2, fusion enthalpy derived from the crystal of the outermost layer was not detected (namely, less than 1 J/g). Although both bending stiffness and elastic modulus when stretched by 10% decreased and this artificial dura mater demonstrated flexibility greater than that produced in Example 3, the coefficient of static friction of the surface thereof was high resulting in poor manipulability. In addition, tensile strength was lower than that of biological dura mater, thereby preventing this artificial dura mater from satisfying the required properties.
The artificial dura mater produced in Comparative Example 3 exhibited fusion enthalpy derived from the crystal of the outermost layer of 19.4, which was higher than that of Example 3, and was found to demonstrate high crystallinity. In this case, this artificial dura mater is extremely hard for use as artificial dura mater, thereby preventing it from satisfying properties that are desired when using as artificial dura mater.

### INDUSTRIAL APPLICABILITY

According to the present invention, it is possible to provide a flexible lactide-ε-caprolactone copolymer for a medical implant that is extremely safe as a result of not containing higher alcohol and is useful for fabricating medical implants requiring flexibility, such as artificial dura mater and artificial blood vessels. In addition, it is possible to provide a method for producing the lactide-ε-caprolactone copolymer for a medical implant, a medical implant and an artificial dura mater which are obtained by using the lactide-ε-caprolactone copolymer for a medical implant.

## Claims

1. A lactide-ε-caprolactone copolymer for a medical implant,
wherein the molar ratio of a lactide to ε-caprolactone (lactide/ε-caprolactone) is 40/60 to 60/40, the weight average molecular weight is 100000 to 500000, a higher alcohol component is not contained therein, and the fusion enthalpy derived from a crystal when formed into a molding by heating and melting is 10 J/g or less.

2. The lactide-ε-caprolactone copolymer for a medical implant according to claim 1,
wherein the fusion enthalpy derived from a crystal when formed into a molding by heating and melting is 1 to 10 J/g.

3. A method for producing a lactide-ε-caprolactone copolymer for a medical implant by copolymerizing a lactide and ε-caprolactone,
wherein a higher alcohol is not added as a polymerization initiator, with a reaction temperature avobe 130°C.

4. A medical implant,
which is obtained by using the lactide-ε-caprolactone copolymer for a medical implant according to claim 1 or 2.

5. The medical implant according to claim 4,
which is in a form of a film, sheet, foamed body, fibrous structure, molding or composite thereof.

6. An artificial dura mater having a multilayer structure composed of a biodegradable/absorbable synthetic polymer,
which comprises an outermost layer composed of the lactide-ε-caprolactone copolymer for a medical implant according to claim 2 and a reinforcing layer having a fibrous structure composed of a biodegradable/absorbable polymer.
